# EUROPEAN PATENT SPECIFICATION

(11) **EP 4 434 431 B1**
(45) Date of publication and mention of the grant of the patent: **02.07.2025**
(21) Application number: 23213932.9
(22) Date of filing: 04.12.2023
(51) Int. Cl.: A61B 1/00, A61B 1/015, A61B 1/307, A61B 18/14, A61B 18/00

(54) **RESECTOSCOPE**
RESEKTOSKOP
RÉSECTOSCOPE

(30) Priority: 23.03.2023 US 202363454160 P
(43) Date of publication of application: 25.09.2024
(73) Proprietor: Olympus Winter & Ibe GmbH, 22045 Hamburg (DE)
(72) Inventor: Brockmann, Christian, 21279 Hollenstedt (DE); Offt, Andreas, 21465 Reinbek (DE); Mauch, Katica, 12529 Schönefeld (DE)
(74) Representative: Hoener, Matthias

(56) References cited:
- WO-A1-97/24074
- GB-A- 2 604 864
- US-A- 5 807 240
- US-A1- 2018 140 351
- US-A1- 2022 249 153
- US-B2- 9 125 550

## Description

The invention relates to a resectoscope according to claim 1.

Resectoscopes of the type described are mainly used for endoscopic applications in urology and gynecology, where they are preferably used for treatment in the area of the bladder or prostate. However, the field of application of these medical instruments is not limited to these areas, but rather also includes the treatment of all organs, preferably the human abdomen.

**In** a standard design, resectoscopes of the generic type have a transporter. For treatment of the diseased organs, the resectoscope, which usually has an elongated shaft formed by an outer tube, is inserted through an opening into the patient's body. Various medical tools for treating and/or examining the patient can be arranged in this shaft or outer tube. For example, in a resectoscope for high-frequency surgery, an electrode that can be subjected to high-frequency alternating current can be inserted into the shaft tube at the distal end of an electrode carrier. For treatments to be performed on patients, such as cutting diseased tissue, the electrode carrier with the electrode is arranged on the resectoscope so that it can be moved relative to the shaft tube.

The electrode carrier is further coupled by its proximal end, preferably movably, to a transport of the resectoscope, by which it can be displaced along a shaft axis to perform the cutting movement of the electrode. The transporter is usually detachably connected to the outer tube. **It** has a longitudinally movable carriage or contact body to which the electrode carrier is coupled for joint longitudinal movement. The actuation or longitudinal displacement of the carriage relative to the transporter is usually performed by an operator. For this purpose, the operator grasps two handles or handle parts which are assigned to different assemblies of the resectoscope so that they can move against each other. According to the state of the art, the movement of the conveyor or of the electrode carrier takes place against or with the spring force of a spring, depending on the design of the conveyor, which in the case of the generic conveyor is usually designed as a leaf spring or as a torsion spring and is arranged between the handles.

Since the aforementioned procedures take place inside the body, the surgeon has no direct view of the area to be operated on. Therefore, a generic resectoscope has an optic or optical unit or lens unit integrated into the resectoscope or shaft. Such optics typically comprise one or more lenses or a light guide disposed within the elongated shaft of the resectoscope. Via the at least one lens, an image is transmitted from within the body through the shaft to the proximal end of the resectoscope. This transmission of image information or light is accomplished either by a plurality of rod lenses arranged axially in series within the shaft, by electronic sensor means such as a CCD chip, or by a light guide means such as a fiber optic also arranged within the shaft of the endoscope.

Prior art documents US 5,807,240, US 2018/140351 and US 9,125,550 disclose resectoscopes according to the preamble of independent claim 1.

In the resectoscope claimed herein, the shaft has an outer tube and an inner tube, wherein a diameter of the inner tube is smaller than a diameter of the outer tube so that the inner tube can be supported in the outer tube. The inner tube guides the optics to the distal end of the shaft.

To ensure that the surgeon has a clear view of the area to be treated via the optics during the treatment, a rinsing fluid is passed through the shaft into the interior of the body during the treatment. This rinsing fluid can be used, for example, to flush away pieces of tissue that are released during resectoscopy. Furthermore, the rinsing fluid serves to remove turbidities caused by blood, for example, from the field of view of the optics. While the rinsing fluid is usually supplied to the inside of the body via the inner tube through an inflow, the contaminated rinsing fluid is aspirated through an outflow. This drain is usually formed by an annular space between the outer tube and the inner tube. A rinsing device or pump can be assigned to the proximal end of the resectoscope or the shaft, so that the rinsing liquid can first be fed into the interior of the body at a predeterminable pressure and, if necessary, can be discharged again through the annular space by means of a slight negative pressure.

For good viewing conditions during surgery, it is essential that the rinsing liquid forms a laminar flow when entering the body cavity, which is at least almost parallel to a longitudinal axis of the shaft or an optical axis of the optics and allows visual inspection of the electrode. As soon as the rinsing fluid does not flow laminarly or even turbulently, the view through the optics can become so poor that an operation cannot be performed. For the formation of a laminar flow, it is particularly important that the rinsing liquid can flow within the inner tube over as long a distance as possible without disturbance. In known resectoscopes, one such disturbance is the electrode carrier which is guided through the inner tube. In particular, turbulence occurs at the tubular supports of the electrode, which can lead to turbulent flow and ultimately to poor visibility conditions in front of the distal end of the resectoscope.

Another disadvantage of known resectoscopes is that the volume flow through the annular space between the inner tube and the outer tube is very limited due to the high frictional forces of the rinsing liquid on the inner wall of the outer tube and the outer wall of the inner tube. In order to increase this volume flow and in particular the outflow of the flushing liquid, it is obvious to reduce the diameter of the inner pipe. However, this would leave less space for the instruments to pass through the inner tube, especially the optics. In addition, a reduction in the cross-section of the inner tube would have a negative effect on the volume flow of the inflow of the rinsing liquid.

Based on this, the problem of the present invention is to create a resectoscope in which the volume flows for the irrigation fluid are optimized and at the same time a particularly laminar flow is formed in front of the distal end of the resectoscope in order to improve the viewing conditions during treatment.

A resectoscope for solving this problem has the features of claim 1. Accordingly, it is provided that the resectoscope has a shaft, at the distal end of which an electrode for manipulating body tissue of a patient is arranged. This shaft is formed by an outer tube in which an inner tube is arranged. Further, the resectoscope has a transporter with a main body, wherein the inner tube is attached to the main body with a proximal end. At least one optic is passable or arranged through the inner tube. Furthermore, the inner tube serves as an inflow and a volume between the outer tube and the inner tube serves as an outflow for a rinsing fluid. The inner tube has different cross-sections at its distal and proximal areas. In this regard, neither the cross-section of the distal area nor the cross-section of the proximal area is circular in shape. This non-circular cross-sectional shape of the inner tube leads to an increase in the volume between the inner tube and the outer tube, which in turn has a positive effect on the flow resistance of the flushing liquid. In addition, this cross-sectional shape allows both an instrument, such as the optics, to be guided through the inner tube and, at the same time, a laminar flow of the rinsing liquid through the inner tube to be achieved.

Preferably, the invention provides that a cross-section of a middle area of the inner tube between the proximal area and the distal area changes continuously along the inner tube from the cross-sectional shape of the proximal area to the cross-sectional shape of the distal area. This continuous transition of the proximal cross-sectional shape to the distal cross-sectional shape results in laminar flow behavior of the fluid. In addition, the electrode carrier with the electrode can be arranged particularly reliably and easily on an outer wall of the inner tube or moved back and forth along the shaft axis

In particular, it is conceivable that the cross-section of the distal and/or proximal area has a waist by means of which the cross-section is divided into two area, namely an upper area, which preferably serves to accommodate the optics, and a lower area, which preferably serves to transport the rinsing liquid. While this waisting shows the inner space of the inner tube to be particularly suitable for receiving the optics and for transporting the rinsing liquid, this shape also results in an increase in the volume between the inner tube and the outer tube. It is conceivable that the upper area is just dimensioned to accommodate the optics. Furthermore, the waist fixes the optic in its position within the inner tube. The waist prevents the optic from slipping into the lower area. Similarly, the waist clearly defines or separates the lower area from the upper area as a flow channel for the rinsing liquid or for holding another instrument. The thickness of the waist or the width of the waist can vary, for example it is conceivable that the two flanks of the waist touch or at least almost touch each other.

One embodiment of the invention may provide that a cross-sectional area of the upper area of the inner tube is equal to, smaller than, or larger than a cross-sectional area of the lower area. Thus, depending on the type of resectoscope or the nature of the optics and any other instruments that may be passed through the inner tube, various cross-sectional shapes for the inner tube are conceivable.

A particular further development of the invention may provide that a cross-sectional area of the upper area is oval or elliptical in shape, wherein a longer axis of symmetry of the cross-sectional area perpendicularly intersects a horizontal plane through the inner tube, so that a channel, in particular a crescent-shaped channel, for the liquid is formed between a circular optic and an upper area of the wall of the inner tube. Furthermore, it is conceivable that the cross-section of the upper area has further widenings in order to increase the free passage area through the inner tube for the flushing liquid. Thus, it has been shown that the formation of the crescent-shaped channel leads to an improved flow behavior of the irrigation fluid, which ultimately results in a very laminar flow in front of the distal end of the resectoscope.

The invention further provides in particular that the cross-section of the distal area of the inner tube has at least one, preferably two, opposing undercuts for fixing an electrode support with an electrode. The tubular supports of the electrode carrier can be coupled to these undercuts. The coupling can be, for example, a push-on or click-on operation. In particular, electrode carriers that are fork-shaped or have at least two parallel carrier tubes can be coupled to the outer wall of the inner tube in a particularly space-saving manner without disturbing the flow behavior of the rinsing liquid. Mounting the electrode support on the inner tube or the conveyor is also particularly reliable and simple, since the electrode support only has to be pushed onto the inner tube from a distal direction. The undercuts fix the electrode carrier in its axial alignment, but it can still be moved back and forth in the axial direction.

It is further conceivable according to the invention that the distal area of the inner tube, in particular a wall of the distal area, has at least one receptacle, in particular an opening, latching means, projections or the like, for the detachable coupling of an electrically insulating attachment, in particular an insulating insert. This attachment prevents the electrode, which is subjected to a high-frequency electrical alternating voltage, from coming into contact with the outer tube. For this purpose, the attachment is arranged at the distal end of the inner tube in such a way that it is located between the electrode and the proximal end in the area of the outer tube. The detachable coupling of the attachment to the inner tube allows it to be easily and reliably disassembled or assembled, for example for cleaning or maintenance purposes.

One possible embodiment of the invention may provide that the length of the distal area is 60 mm to 210 mm, preferably 90 mm to 190 mm, 90 mm to 120 mm or 160 mm to 190 mm, . The length of the proximal area may be 24 mm to 200 mm, preferably 90 mm to 170 mm . The length of the middle area is dimensioned accordingly. In addition, it should be mentioned that the aforementioned areas can also have other lengths.

Preferably, it is further conceivable that the cross-section of the proximal area of the inner tube has an upper area and a lower area, the upper area being round, preferably circular, and the lower area having two opposite straight and parallel or concave cross-sectional flanks. These straight or concave cross-sectional flanks in the proximal area prove to be particularly advantageous for guiding the proximal ends of the electrode support to the main body of the conveyor. Since this cross-sectional shape is limited to the proximal area of the inner tube, this shape has no influence on the formation of laminar flow in the distal area of the inner tube. With slightly concave flanks, it is also possible to create a larger gap between the electrode supports and the tube without restricting the flow cross section too much. Unlike straight flanks, concave flanks have a preferred direction when pressed into a main body. This means that they bend inward into the flow cross-section when upset, while random inward or outward bending can occur with straight flanks. In addition to this keyhole-like shape of the cross-section of the proximal area of the inner tube, it is also conceivable for this area to have other shapes which serve equally to bring the electrode support particularly advantageously close to the main body and at the same time serve to transport the optics and the rinsing liquid to the distal end.

It is further conceivable that the upper areas of the proximal and distal cross-sections have the same cross-sectional shape and/or that the lower areas of the proximal and distal cross-sections have the same cross-sectional shape. Due to this flexible design of the cross-sectional shapes, different embodiments of the inner tube for different application purposes are conceivable.

Another preferred embodiment of the invention may provide that a proximal end of the inner tube is fixedly connected to the main body of the conveyor, preferably pressed and welded or plugged and welded or joined together. In particular, it is conceivable that a proximal end of the inner tube has a press-fit section with which the inner tube can be pressed to the main body. One possible method for pressing is hydroforming (hydroforming). In this process, the inner tube with the proximal end or the press-fit section is guided into the main body of the conveyor and subjected to high pressure. By applying the high pressure, the inner tube or the press-fit section expands and presses into the main body. This allows a very stable connection to be made between the inner tube and the main body in a simple and inexpensive manner. When pressing in using hydroforming, welding can be dispensed with if necessary by creating a form fit between the tube and the main body (undercut in the main body into which the tube is radially pressed).

For pressing the inner tube into the main body, it may be provided that the cross-section of the press-fit section has an upper area and a lower area, the upper area being round, preferably circular, and the lower area having two opposite concave cross-sectional flanks which are connected to one another by a round arc. It has been shown that these concave cross-sectional flanks are particularly suitable for guiding the inner tube or the pressed-in section into the main body and pressing it there. In addition, this cross-sectional shape offers the advantages already further elaborated above. According to the invention, it is conceivable that the length of this pressed-in section is 5 mm to 20 mm, preferably 10 mm.

A preferred embodiment of the invention is explained in more detail below with reference to the drawing. In this show:
- Fig. 1: an illustration of a resectoscope,
- Fig. 2: a schematic representation of an inner tube,
- Fig. 3: an illustration of a cross-section of a distal area of the shaft,
- Fig. 4: an illustration of a cross-section of a proximal area of the stem,
- Fig. 5: an illustration of a cross-section of a proximal area of the stem,
- Fig. 6: an illustration of a cross-section of the inner tube, and
- Fig. 7: an illustration of a cross-section of a press-fit section.

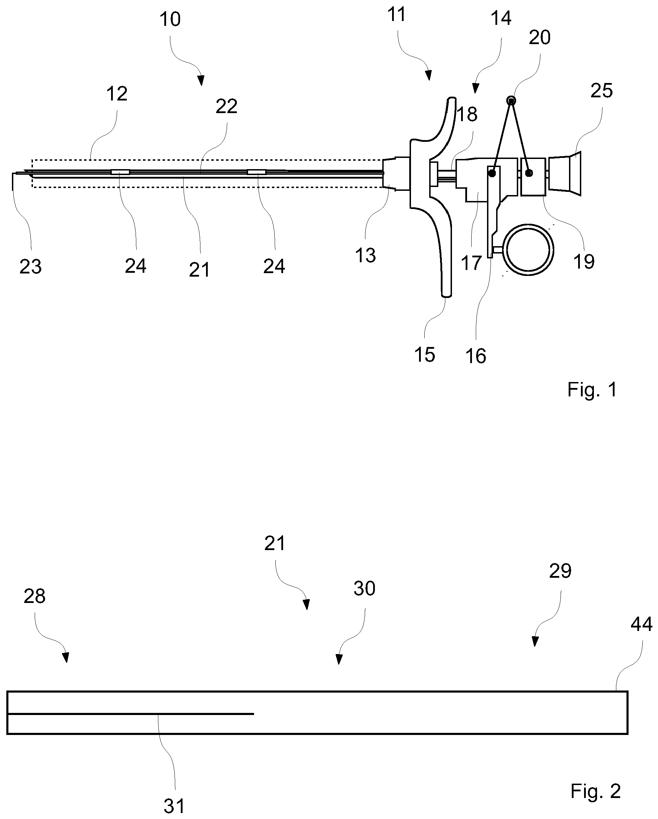

A possible embodiment of a handheld electrosurgical device, namely a resectoscope 10, is shown in highly schematized form in Fig. 1. The resectoscope 10 has a transporter 11 with an elongated, tube-like shaft or outer tube 12. This outer tube 12 is shown hatched in Fig. 1 and is attachable at a proximal end to a main body 13 of the transporter 11.

In addition to the main body 13, the transporter 11 has a handle unit 14. The transporter 11 can have a, in particular detachable, handle 15. In the embodiment of the conveyor 11 shown here, a gripping means 16 is assigned to a contact body 17. It is conceivable that the gripping means 16 is screwed tightly to the contact body 17.

The contact body 17 is slidably guided on a tubular optics guide 18. Since the contact body 17 can be moved back and forth on the optics guide 18 along a longitudinal direction of the resectoscope 10 or a longitudinal axis of the outer tube 12, the contact body 17 is also referred to as a carriage. While a distal end of the optics guide 18 is connectable to the main body 13, a optics guide plate 19 is attached to a proximal end of the optics guide 18. The tube-like optics guide 18 extends through the optics guide plate 19 so that the optics guide 18 is accessible from proximally.

The gripping means 16 or the contact body 17 are connected to the optics guide plate 19 via a spring element 20. This spring element 20 can be a tension spring or a compression spring, depending on the design of the conveyor 11.

Starting from the main body 13, a tubular inner tube 21 extends in the distal direction. An electrode carrier 22 extends parallel to the inner tube 21. This electrode carrier 22 is guided through the main body 13 and is detachably coupled mechanically to the contact body 17 with at least one proximal contact. At a distal end, the electrode carrier 22 has an electrode 23. An electrical RF voltage can be applied to this electrode 23. By means of a thermal plasma forming at the electrode 23, the diseased tissue can be manipulated or cut. To do this, the operator moves the gripping means 16 relative to the conveyor 11. To stabilize the electrode carrier 22, this can be guided on the inner tube 21 by guides 24.

To perform the operation, a rod-like optic 26 is guided through the inner tube 21 or through the optic guide 18. A distal end of this optic 26, which is not visible here, is directed toward the electrode 23 so that the surgeon has a view of the manipulation of the tissue. This optic 26 may be a rod lens system or an optical fiber. At the proximal end of the optic 26 is a eyepiece 25 or camera, as shown in Fig. 1.

A ring-like insulating attachment, not shown, is removably associated with the distal end of the inner tube 12. This insulating attachment serves to electrically insulate the electrode 23 from the outer tube 12. In order for the insulating attachment to be electrically insulating, it may be made of plastic or ceramic.

To ensure that the surgeon's view of the area to be operated on is not obstructed by blood or tissue particles during the operation, this area can be supplied with a rinsing liquid. For this purpose, the rinsing liquid is passed through the inner tube 21 and emerges from the distal end of the inner tube 21 parallel to the optics 26. In order to remove the rinsing liquid from the interior of the body again, it can be sucked out through a volume 27 between the inner tube 21 and the outer tube 12 by a suction device or pump that is not shown. By generating a negative pressure, the flushing liquid can be removed via the volume 27.

In Fig. 2, the inner tube 21 is shown in a highly schematized form. This inner tube 21 has a distal area 28, a proximal area 29, and a middle area 30. The essence of the present invention is that a cross-section of the distal area 28 of the inner tube 21 is different from a cross-section of the proximal area 29. The cross sections continuously merge into each other in the middle area 30. In addition, the inner tube 21 and in particular, as shown in Fig. 2, the distal area 28 has an undercut 31. This undercut 31, which is also formed on the opposite wall half of the inner tube 21 not visible in Fig. 2, serves to accommodate the electrode carrier 22 with its guides 24. In this case, the electrode carrier 22 with the two tubes 32 and the guides 24 is pushed over the inner tube 21 in such a way that it is radially fixed and axially movable in the undercut 31. Embodiments of the inner tube 21 in which the undercut 31 extends over the entire length of the inner tube 21 are also conceivable.

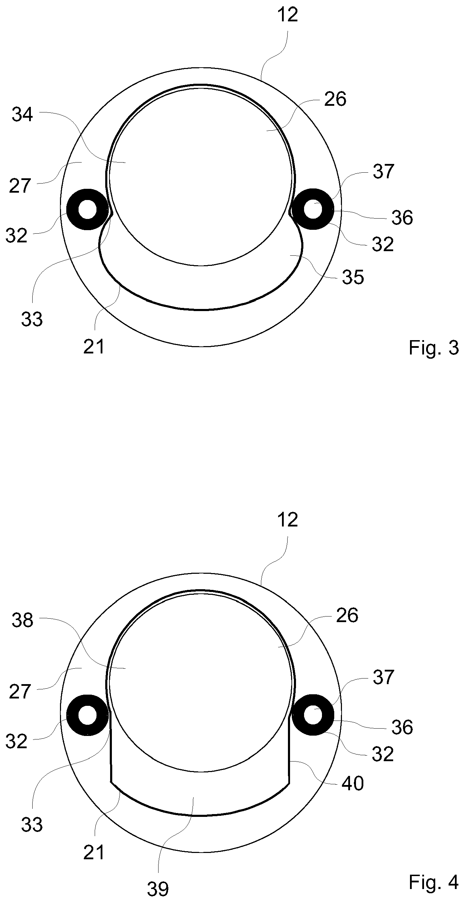

In Fig. 3, the cross-section of the distal area 28 of the inner tube 21 is shown. Also shown is the cross-section of the outer tube 12, the optics 26, and the two tubes 32 of the electrode support 22. From this schematic illustration, it can be seen that the cross-section of the distal area 28 of the inner tube 21 has a waist 33 or is waisted. This waist 33 separates the cross-section into an upper area 34 and a lower area 35. As can already be seen from Fig. 3, the upper area 34 is circular in shape, whereas the lower area 35 is oval. The circular upper area 34 is just sized to accommodate the optic 26. The lower area 35 of the inner tube 21 is designed to allow the rinsing fluid to flow from the proximal area 29 to the electrode 23. Due to the available volume and the external mounting of the electrode support 22, the rinsing liquid can flow through the lower area 35 almost without interference, at least in the distal area 28 of the inner tube 21, so that the flow already behaves laminarly in the inner tube 21 and thus also outside the tube 21. The laminar flow is particularly advantageous for the viewing conditions in front of the optics 26.

Another advantage resulting from the waisted shape of the distal area 28 of the inner tube 21 is that the volume 27 between the outer tube 12 and the inner tube 21 is increased. Compared to a circular inner tube, the waist 33 creates space through which the flushing liquid can flow back to the proximal area 29. In addition, the tubes 32 of the electrode carrier 22 can be guided in this area. The electrode support 22 and/or the tubes 32 are shown here in section. Both the outer tube 32, the electrical insulation 36 and the electrical conductor 37 are visible.

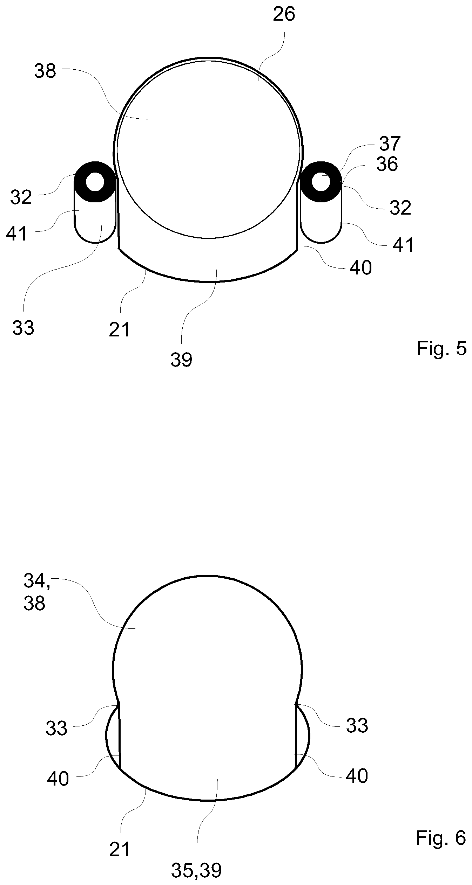

As mentioned above, the cross-section of the proximal area 29 of the inner tube 21 has a different shape. This shape is shown schematically in Fig. 4. It can be seen that the proximal area 29 of the inner tube 21 also has an upper area 38 and a middle area 30. While the upper area 38 is formed in the same way as the upper area 34, namely to accommodate the optics 26, the lower area 39 has two straight as well as parallel cross-sectional flanks 40; alternatively, it is also conceivable that these flanks 40 are concave. The straight flanks 40 prove to be particularly advantageous for guiding the tubes 32 in the proximal area 29, where the tubes 32 or the electrode support 22 describe a downwardly directed S-stroke 41, as indicated in Fig. 5. This S- stroke 41 displaces the plane in which the electrode support 22 is located so that the proximal ends of the tubes 32 can be guided through the main body 13 of the conveyor 11.

In Fig. 6, a cross-section or view along the entire length of the inner tube 21 is shown. From this, it can be seen that the cross-sectional shape of the distal aera 28 continuously transitions into the cross-sectional shape of the proximal aera 29 via the middle aera 30. The length of the aeras 28 to 30 may be different for different embodiments of the resectoscope 10. For example, it is conceivable that the length of distal aera 28 is 60 mm to 210 mm, preferably 90 mm to 190 mm, 90 mm to 120 mm, or 160 mm to 190 mm, and the length of proximal aera 29 is 24 mm to 200 mm, preferably 90 mm to 170 mm. The length of the middle aera 30 is correspondingly dimensioned.

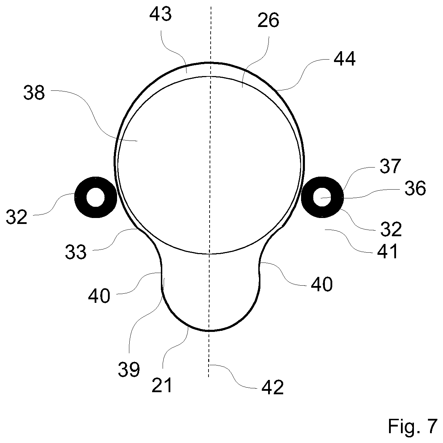

As an alternative to the straight cross-sectional flanks 40, it is also conceivable that these flanks are concave. Such an embodiment is shown in Fig. 7. This concave design of the cross-sectional flanks 40 reduces the size of the lower aera 39. Similarly, this increases the volume 27 between the outer tube 12 and the inner tube 21. Another advantage of this cross-sectional shape is the mounting of the inner tube 21 in the main body 13. To mount the inner tube 21 in the main body 13, the proximal aera 29 must first be pushed into the main body 13. Due to the shape of the cross-section shown in Fig. 7, the inner tube 21 can be better fixed in the main body 13. From Fig. 7, another advantageous feature also becomes clear and that is that it is oval or elliptical in shape. Here, the oval or elliptical shape is erected such that a longer axis of symmetry 42 of the cross-sectional area perpendicularly intersects a horizontal plane through the inner tube 21. As a result, a crescent-shaped channel 43 is formed between the optics 26 and the undercut 31 of the inner tube 21. This channel 43 allows additional flushing fluid to flow through the inner tube 32.

To fix the inner tube 21 in the main body 13, it is provided that the proximal area 29 is connected to the main body 13. A particularly advantageous method for connecting the inner tube 21 to the main body 13 is hydroforming. In this process, a rear section of the proximal aera 29, the so-called press-fit section 44, which has, for example, the cross-sectional shape shown in Fig. 7, is subjected to high pressure. As a result, the wall of the inner tube 21 is pressed into the opening of the main body 13. Subsequently, the components are still welded together. Other processes are also conceivable for joining the inner tube 21 to the main body 13.

### List of Reference Numerals:

| | | | |
|---|---|---|---|
| 10 | Resectoscope | 36 | Insulation |
| 11 | Transporter | 37 | Conductor |
| 12 | Outer tube | 38 | Upper area |
| 13 | Main body | 39 | Lower area |
| 14 | Handle unit | 40 | Cross section flank |
| 15 | Handle | 41 | S-stroke |
| 16 | Gripping means | 42 | Symmetry axis |
| 17 | Contact body | 43 | Channel |
| 18 | Optics guide | 44 | Press-fit section |
| 19 | Optics guide plate | | |
| 20 | Spring element | | |
| 21 | Inner tube | | |
| 22 | Electrode carrier | | |
| 23 | Electrode | | |
| 24 | Guiding element | | |
| 25 | Ocular | | |
| 26 | Optical system | | |
| 27 | Volume | | |
| 28 | Distal area | | |
| 29 | Proximal area | | |
| 30 | Middle area | | |
| 31 | Undercut | | |
| 32 | Pipe | | |
| 33 | Waist | | |
| 34 | Upper area | | |
| 35 | Lower area | | |

## Claims

1. Resectoscope (10) with an outer tube (12) and an inner tube (21) arranged in the outer tube (12) and with a transporter (11) comprising a main body (13), wherein the inner tube (21) is attached to the main body (13) with a proximal end, wherein at least one optical system (26) is arranged in the inner tube (21) and the inner tube (21) is formed as an inflow and a volume (27) between the outer tube (12) and the inner tube (21) is formed as an outflow for a rinsing liquid, wherein neither the cross section of the distal area (28) nor the cross section of the proximal area (29) is circular,
**characterized in that** a cross section of a distal area (28) of the inner tube (21) is different from a cross section of a proximal area (29) of the inner tube (21).

2. Resectoscope (10) according to claim 1, **characterized in that** a cross-section of a middle area (30) of the inner tube (21) between the proximal and distal area (28, 29) changes continuously along the inner tube (21) from the cross-sectional shape of the proximal area (29) to the cross-sectional shape of the distal area (28).

3. Resectoscope (10) according to claim 1 or 2, **characterized in that** the cross-section of the distal and/or the proximal area (28, 29) has a waist (33) by which the cross-section is divided into two areas, namely an upper area (34, 38), which preferably serves to receive an optic (26), and a lower area (35, 39), which preferably serves to transport a rinsing liquid.

4. Resectoscope (10) according to claim 3, **characterized in that** a cross-sectional area of the upper area (34, 38) is equal to, smaller than, or larger than a cross-sectional area of the lower area (35, 39).

5. Resectoscope (10) according to claim 3 or 4, **characterized in that** a cross-sectional area of the upper area (34, 38) is oval or elliptical in shape, wherein a longer axis of symmetry (42) of the cross-sectional area perpendicularly intersects a horizontal plane through the inner tube (21), so that a channel (43), in particular a sickle-shaped channel, for the rinsing liquid is formed between a circular optic (26) and an upper portion of a wall of the inner tube (21).

6. resectoscope (10) according to one of the previous claims, **characterized in that** the cross-section of the distal area (28) of the inner tube (21) has at least one, preferably two, opposing undercuts (31) for fixing an electrode carrier (22) with an electrode (23).

7. resectoscope (10) according to one of the previous claims, **characterized in that** the distal area (28) of the inner tube (21), in particular a wall of the distal area (28), has at least one receptacle, in particular openings, latching means, projections or the like, for releasable coupling of an electrically insulating attachment, in particular an insulating attachment.

8. Resectoscope (10) according to any one of the preceding claims, **characterized in that** the length of the distal area (28) is 60 mm to 210 mm, preferably 90 mm to 190 mm, 90 mm to 120 mm or 160 mm to 190 mm.

9. Resectoscope (10) according to any one of the preceding claims, **characterized in that** the cross-section of the proximal area (29) of the inner tube (21) has an upper area (34, 38) and a lower area (35, 39), the upper area (34, 38) being round, preferably circular, and the lower area (35, 39) having two opposite straight and parallel or concave cross-sectional flanks (40).

10. Resectoscope (10) according to any of the preceding claims, **characterized in that** the length of the proximal area (29) is 24 mm to 200 mm, preferably 90 mm to 170 mm.

11. Resectoscope (10) according to any one of the preceding claims, **characterized in that** the upper areas (34, 38) of the proximal and distal areas (28, 29) have the same cross-sectional shape and/or that the lower areas (35, 39) of the proximal and distal areas (28, 29) have the same cross-sectional shape.

12. Resectoscope (10) according to any one of the preceding claims, **characterized in that** a proximal end of the inner tube (21) is firmly connected, preferably pressed, or joined to the main body (13) of the transporter (11).

13. Resectoscope (10) according to any one of the preceding claims, **characterized in that** a proximal end of the inner tube has a press-fit section (44) by means of which the inner tube (21) can be pressed to the main body (13).

14. Resectoscope (10) according to claim 13, **characterized in that** the cross-section of the press-fit section (44) has an upper area (34, 38) and a lower area (35, 39), the upper area (34, 38) being round, preferably circular, and the lower area (35, 39) having two opposite concave cross-sectional flanks (40).

15. Resectoscope (10) according to claim 13 or 14, **characterized in that** the length of the press-fit section (44) is 5 mm to 20 mm, preferably 10 mm.

## Patentansprüche

1. Resektoskop (10) mit einem Außenrohr (12) und einem in dem Außenrohr (12) angeordneten Innenrohr (21) und mit einem Transporteur (11), der einen Hauptkörper (13) umfasst, wobei das Innenrohr (21) mit einem proximalen Ende an dem Hauptkörper (13) befestigt ist, wobei in dem Innenrohr (21) mindestens ein optisches System (26) angeordnet ist und das Innenrohr (21) als Zufluss und ein Volumen (27) zwischen dem Außenrohr (12) und dem Innenrohr (21) als Abfluss für eine Spülflüssigkeit ausgebildet sind, wobei weder der Querschnitt des distalen Bereichs (28) noch der Querschnitt des proximalen Bereichs (29) kreisförmig sind,
**dadurch gekennzeichnet, dass** ein Querschnitt eines distalen Bereichs (28) des Innenrohrs (21) sich von einem Querschnitt eines proximalen Bereichs (29) des Innenrohrs (21) unterscheidet.

2. Resektoskop (10) nach Anspruch 1, **dadurch gekennzeichnet, dass** sich ein Querschnitt eines mittleren Bereichs (30) des Innenrohrs (21) zwischen dem proximalen und dem distalen Bereich (28, 29) entlang des Innenrohrs (21) kontinuierlich von der Querschnittsform des proximalen Bereichs (29) zu der Querschnittsform des distalen Bereichs (28) ändert.

3. Resektoskop (10) nach Anspruch 1 oder 2, **dadurch gekennzeichnet, dass** der Querschnitt des distalen und/oder des proximalen Bereichs (28, 29) eine Taille (33) aufweist, durch die der Querschnitt in zwei Bereiche aufgeteilt wird, nämlich in einen oberen Bereich (34, 38), der vorzugsweise zur Aufnahme einer Optik (26) dient, und einen unteren Bereich (35, 39), der vorzugsweise zum Transport einer Spülflüssigkeit dient.

4. Resektoskop (10) nach Anspruch 3, **dadurch gekennzeichnet, dass** eine Querschnittsfläche des oberen Bereichs (34, 38) gleich, kleiner oder größer als eine Querschnittsfläche des unteren Bereichs (35, 39) ist.

5. Resektoskop (10) nach Anspruch 3 oder 4, **dadurch gekennzeichnet, dass** eine Querschnittsfläche des oberen Bereichs (34, 38) oval oder elliptisch geformt ist, wobei eine längere Symmetrieachse (42) der Querschnittsfläche eine horizontale Ebene durch das Innenrohr (21) senkrecht schneidet, so dass zwischen einer kreisförmigen Optik (26) und einem oberen Abschnitt einer Wandung des Innenrohrs (21) ein Kanal (43), insbesondere ein sichelförmiger Kanal, für die Spülflüssigkeit ausgebildet wird.

6. Resektoskop (10) nach einem der vorherigen Ansprüche, **dadurch gekennzeichnet, dass** der Querschnitt des distalen Bereichs (28) des Innenrohrs (21) mindestens einen, vorzugsweise zwei gegenüberliegende, Hinterschnitte (31) zur Fixierung eines Elektrodenträgers (22) mit einer Elektrode (23) aufweist.

7. Resektoskop (10) nach einem der vorherigen Ansprüche, **dadurch gekennzeichnet, dass** der distale Bereich (28) des Innenrohrs (21), insbesondere eine Wandung des distalen Bereichs (28), mindestens eine Aufnahme, insbesondere Öffnungen, Rastmittel, Vorsprünge oder dergleichen, zur lösbaren Kopplung eines elektrisch isolierenden Aufsatzes, insbesondere eines Isolieraufsatzes, aufweist.

8. Resektoskop (10) nach einem der vorherigen Ansprüche, **dadurch gekennzeichnet, dass** die Länge des distalen Bereichs (28) 60 mm bis 210 mm, vorzugsweise 90 mm bis 190 mm, 90 mm bis 120 mm oder 160 mm bis 190 mm, beträgt.

9. Resektoskop (10) nach einem der vorherigen Ansprüche, **dadurch gekennzeichnet, dass** der Querschnitt des proximalen Bereichs (29) des Innenrohrs (21) einen oberen Bereich (34, 38) und einen unteren Bereich (35, 39) aufweist, wobei der obere Bereich (34, 38) rund, vorzugsweise kreisartig, ist und der untere Bereich (35, 39) zwei gegenüberliegende gerade und parallele oder konkave Querschnittsflanken (40) aufweist.

10. Resektoskop (10) nach einem der vorherigen Ansprüche, **dadurch gekennzeichnet, dass** die Länge des proximalen Bereichs (29) 24 mm bis 200 mm, vorzugsweise 90 mm bis 170 mm, beträgt.

11. Resektoskop (10) nach einem der vorherigen Ansprüche, **dadurch gekennzeichnet, dass** die oberen Bereiche (34, 38) des proximalen und des distalen Bereichs (28, 29) die gleiche Querschnittsform aufweisen und/oder dass die unteren Bereiche (35, 39) des proximalen und des distalen Bereichs (28, 29) die gleiche Querschnittsform aufweisen.

12. Resektoskop (10) nach einem der vorherigen Ansprüche, **dadurch gekennzeichnet, dass** ein proximales Ende des Innenrohrs (21) mit dem Hauptkörper (13) des Transporteurs (11) fest verbunden, vorzugsweise verpresst, oder zusammengefügt ist.

13. Resektoskop (10) nach einem der vorherigen Ansprüche, **dadurch gekennzeichnet, dass** ein proximales Ende des Innenrohrs einen Einpressabschnitt (44) aufweist, mit dem das Innenrohr (21) mit dem Hauptkörper (13) verpressbar ist.

14. Resektoskop (10) nach Anspruch 13, **dadurch gekennzeichnet, dass** der Querschnitt des Einpressabschitts (44) einen oberen Bereich (34, 38) und einen unteren Bereich (35, 39) aufweist, wobei der obere Bereich (34, 38) rund, vorzugsweise kreisartig, ist und der untere Bereich (35, 39) zwei gegenüberliegende konkave Querschnittsflanken (40) aufweist.

15. Resektoskop (10) nach Anspruch 13 oder 14, **dadurch gekennzeichnet, dass** die Länge des Einpressabschnitts (44) 5 mm bis 20 mm, vorzugsweise 10 mm, beträgt.

## Revendications

1. Résectoscope (10) avec un tube extérieur (12) et un tube intérieur (21) agencé dans le tube extérieur (12) et avec un transporteur (11) comprenant un corps principal (13), le tube intérieur (21) étant fixé au corps principal (13) avec une extrémité proximale, au moins un système optique (26) étant agencé dans le tube intérieur (21) et le tube intérieur (21) étant formé comme un flux entrant et un volume (27) entre le tube extérieur (12) et le tube intérieur (21) étant formé comme un flux sortant pour un liquide de rinçage, ni la section transversale de la zone distale (28) ni la section transversale de la zone proximale (29) n'étant circulaires,
**caractérisé en ce qu'**une section transversale de la zone distale (28) du tube intérieur (21) est différente d'une section transversale de la zone proximale (29) du tube intérieur (21).

2. Résectoscope (10) selon la revendication 1, **caractérisé en ce qu'**une section transversale d'une zone médiane (30) du tube intérieur (21) entre les zones proximale et distale (28, 29) change continuellement le long du tube intérieur (21) de la forme de la section transversale de la zone proximale (29) à la forme de la section transversale de la zone distale (28).

3. Résectoscope (10) selon la revendication 1 ou 2, **caractérisé en ce que** la section transversale de la zone distale et/ou de la zone proximale (28, 29) présente une taille (33) par laquelle la section transversale est divisée en deux zones, à savoir une zone supérieure (34, 38), qui sert de préférence à recevoir une optique (26), et une zone inférieure (35, 39), qui sert de préférence à transporter un liquide de rinçage.

4. Résectoscope (10) selon la revendication 3, **caractérisé en ce qu'**une section transversale de la zone supérieure (34, 38) est égale, inférieure ou supérieure à une section transversale de la zone inférieure (35, 39).

5. Résectoscope (10) selon la revendication 3 ou 4, **caractérisé en ce qu'**une section transversale de la zone supérieure (34, 38) est de forme ovale ou elliptique, un axe de symétrie plus long (42) de la section transversale coupant perpendiculairement un plan horizontal à travers le tube intérieur (21), de sorte qu'un canal (43), en particulier un canal en forme de faucille, pour le liquide de rinçage est formé entre une optique circulaire (26) et une partie supérieure d'une paroi du tube intérieur (21).

6. Résectoscope (10) selon l'une des revendications précédentes, **caractérisé en ce que** la section transversale de la zone distale (28) du tube intérieur (21) présente au moins une, de préférence deux, contre-dépouilles (31) opposées pour la fixation d'un porte-électrode (22) avec une électrode (23).

7. Résectoscope (10) selon l'une des revendications précédentes, **caractérisé en ce que** la zone distale (28) du tube intérieur (21), en particulier une paroi de la zone distale (28), présente au moins un réceptacle, en particulier des ouvertures, des moyens de verrouillage, des saillies ou similaires, pour le couplage amovible d'un accessoire électriquement isolant, en particulier un accessoire isolant.

8. Résectoscope (10) selon l'une quelconque des revendications précédentes, **caractérisé en ce que** la longueur de la zone distale (28) est comprise entre 60 mm et 210 mm, de préférence entre 90 mm et 190 mm, entre 90 mm et 120 mm ou entre 160 mm et 190 mm.

9. Résectoscope (10) selon l'une quelconque des revendications précédentes, **caractérisé en ce que** la section transversale de la zone proximale (29) du tube intérieur (21) présente une zone supérieure (34, 38) et une zone inférieure (35, 39), la zone supérieure (34, 38) étant arrondie, de préférence circulaire, et la zone inférieure (35, 39) présentant deux flancs opposés de section droite et parallèle ou concave (40).

10. Résectoscope (10) selon l'une quelconque des revendications précédentes, **caractérisé en ce que** la longueur de la zone proximale (29) est comprise entre 24 mm et 200 mm, de préférence entre 90 mm et 170 mm.

11. Résectoscope (10) selon l'une quelconque des revendications précédentes, **caractérisé en ce que** les zones supérieures (34, 38) des zones proximale et distale (28, 29) ont la même forme de section transversale et/ou **en ce que** les zones inférieures (35, 39) des zones proximale et distale (28, 29) ont la même forme de section transversale.

12. Résectoscope (10) selon l'une quelconque des revendications précédentes, **caractérisé en ce qu'**une extrémité proximale du tube intérieur (21) est fermement reliée, de préférence pressée, ou jointe au corps principal (13) du transporteur (11).

13. Résectoscope (10) selon l'une quelconque des revendications précédentes, **caractérisé en ce qu'**une extrémité proximale du tube intérieur comporte une section à sertir (44) au moyen de laquelle le tube intérieur (21) peut être pressé sur le corps principal (13).

14. Résectoscope (10) selon la revendication 13, **caractérisé en ce que** la section transversale de la section d'ajustement (44) présente une zone supérieure (34, 38) et une zone inférieure (35, 39), la zone supérieure (34, 38) étant arrondie, de préférence circulaire, et la zone inférieure (35, 39) présentant deux flancs opposés de section transversale concave (40).

15. Résectoscope (10) selon la revendication 13 ou 14, **caractérisé en ce que** la longueur de la section d'ajustement (44) est comprise entre 5 mm et 20 mm, de préférence 10 mm.
